# NEUE EUROPÄISCHE PATENTSCHRIFT

(11) **EP 0 396 650 B2**
(45) Veröffentlichungstag und Bekanntmachung des Hinweises auf die Entscheidung über den Einspruch: **12.04.1995**
(45) Hinweis auf die Patenterteilung: 14.10.1992
(21) Anmeldenummer: 89909146.6
(22) Anmeldetag: 22.08.1989
(51) Int. Cl.: B01J 12/00, B01J 35/04, B01D 3/00, C07C 41/06, C07C 51/215, C07C 57/145

(54) **VORRICHTUNG ZUR DURCHFÜHRUNG KATALYSIERTER REAKTIONEN**
DEVICE FOR CARRYING OUT CATALYTIC REACTIONS
DISPOSITIF DE MISE EN UVRE DE REACTIONS CATALYSEES

(30) Priorität: 02.09.1988 CH 3295/88; 17.02.1989 CH 577/89
(43) Veröffentlichungstag der Anmeldung: 14.11.1990
(73) Patentinhaber: GEBRÜDER SULZER AKTIENGESELLSCHAFT, CH-8401 Winterthur (CH)
(72) Erfinder: SHELDEN, Ronald, CH-8400 Winterthur (CH); STRINGARO, Jean-Paul, CH-8180 Bülach (CH)
(74) Vertreter: Dipl.-Phys.Dr. Manitz Dipl.-Ing. Finsterwald Dipl.-Ing. Grämkow Dipl.Chem.Dr. Heyn Dipl.Phys. Rotermund Morgan, B.Sc.(Phys.)
(86) Internationale Anmeldenummer: CH8900150
(87) Internationale Veröffentlichungsnummer: WO9002603

(56) Entgegenhaltungen:
- EP-A- 0 008 860
- EP-A- 0 210 614
- BE-A- 815 237
- CH-A- 398 503
- DE-A- 2 037 194
- GB-A- 1 186 647
- GB-A- 1 471 442
- GB-A- 1 569 828
- GB-A- 1 604 361
- US-A- 3 727 384
- US-A- 3 926 851
- US-A- 4 443 559
- US-A- 4 455 339
- US-A- 4 497 751
- US-A- 4 497 752
- US-A- 4 497 753
- US-A- 4 731 229
- US-A- 4 744 928
- Patent Abstracts of Japan, Band 1, Nr. 111, (C-027), 26. September 1977
- Chemical Processing, Feb. 1987, S. 27-31

## Beschreibung

Die Erfindung betrifft eine Vorrichtung zur Durchführung katalysierter Reaktionen, sowie auf die Verwendung derartiger Vorrichtungen.

Ein wesentliches Anwendungsgebiet der Erfindung sind katalytische Festbettreaktoren, beispielsweise für Oxydations- oder Hydrierungsreaktionen.

Bei bekannten Ausführungsformen derartiger Reaktoren werden im Reaktionsraum Schüttungen von festen Katalysatorpartikelchen, die z.B. kugel- oder pelletförmig ausgebildet sind, untergebracht, welche von den Reaktanden umströmt werden.

Bei den so ausgebildeten Reaktoren entstehen einerseits hohe Druckabfälle, andererseits findet über dem Reaktionsquerschnitt keine homogene Temperaturverteilung statt. Ein weiterer Nachteil besteht darin, dass keine einheitliche Konzentrationsverteilung über den Reaktorquerschnitt erfolgt, so dass die Ausbeute der gewünschten Endprodukte nicht optimal ist.

Schliesslich verläuft eine grosse Anzahl der üblichen chemischen Reaktionen exotherm, so dass im Reaktorbett örtliche Ueberhitzungen (sog. Hot Spots) entstehen, welche die Lebensdauer der Katalysatormaterialien begrenzen und insbesondere bei Oxidationsreaktionen die Selektivität herabsetzen.

Um die Auswirkungen der hierbei entstehenden örtlichen Ueberhitzungen in Grenzen zu halten, d.h. um eine verbesserte Abführung der Wärme an die Reaktorwandungen zu erreichen, ist es üblich, im Reaktionsraum eine Anzahl von Rohren mit Katalysatorschüttungen anzuordnen, welche von einem wärmeaufnehmenden Mittel umströmt werden. Jedoch ist eine solche Ausführungsweise äusserst kostenintensiv im Betrieb und herstellungsmässig aufwendig.

Bildet man die Lagen der Vorrichtungen selbst aus Katalysatormaterial aus, so setzt dieses voraus, dass das Material auf einfache Weise in die gewünschte Form gebracht werden kann. Ausserdem sind solche Materialien unter Umständen sehr kostenaufwendig.

Aus der Literatur sind verschiedene Vorrichtungen bekannt, welche einzelne Merkmale zur Verbesserung von Kontakteigenschaften bzw. der Katalysatorwirkung aufweisen:

Die BE 815 237 offenbart eine Kontaktvorrichtung für Reaktionen zwischen Fluiden und Feststoffen mit parallelen Kanälen, die DE-A-2 037 194 eine Katalysatorvorrichtung mit gasdurchlässigen Tafeln und das Patent Abstract JP-A-52075657 eine Abgaskatalysatorvorrichtung mit parallelen Gaskanälen, während die EP-A-0 008 860 eine katalytische Destillationsvorrichtung mit festem Katalysator in einer gewickelten Gittertaschenstruktur beschreibt. All diese Vorrichtungen und Anordnungen ergeben jedoch keine guten Mischungs- und Homogenisierungseigenschaften. Sie weisen damit immer noch die erwähnten Nachteile bezüglich katalytischer Wirksamkeit. Effizienz und Lebensdauer auf.

Die Erfindung hat es sich zur Aufgabe gemacht, eine Vorrichtung zu schaffen, die eine vorteilhafte Ausnutzung der Katalysatormaterialien und lange Lebensdauer bei der Durchführung von katalysierten Reaktionen ermöglicht, sowie die Verwendung eines breiten Spektrums von Katalysatormaterialien gestattet.

Diese Aufgabe wird erfindungsgemäss durch die Merkmale von Anspruch 1 gelöst. Die abhängigen Ansprüche beziehen sich auf besonders vorteilhafte Ausführungsformen der Erfindung.

Wesentliche Vorteile der Erfindung sind homogene Temperaturverteilung, Konzentrationsausgleich über den querschnitt, enge Verweilzeitverteilung und sehr wenig bis keine Rückvermischung.

Der homogene Temperaturausgleich und der günstige Warmeübergang an den Reaktionwandungen ist insbesondere massgebend für eine lange Lebensdauer der Katalysatormaterialien und für hohe Produktausbeute und Selektivität. Insbesondere ist dieser Effekt wesentlich bei exotherm verlaufenden Reaktionen, da die hierbei entstehende Wärme an überhitzten Stellen rasch an die Reaktorwandungen weggeführt werden kann. Dies führt zu erhöhter Betriebssicherheit (kein runaway).

Von wesentlicher Bedeutung ist es auch, dass aufgrund dieser gleichmässigen Temperaturverteilung und des über den Reaktorquerschnitt erfolgenden Konzentrationsausgleiches das gewünschte Reaktionsprodukt in grosser Menge und Güte gewonnen werden kann und die unreagierten Edukte nur in relativ kleiner Menge entstehen.

Die mit erfindungsgemässen Vorrichtungen ausgerüsteten Festbettreaktoren, können im Vergleich zu bekannten Festbettreaktoren, bei gleichem Durchsatz, mit einer wesentlich geringeren Anzahl von Einzelrohren ausgebildet werden. Gegebenenfalls reicht eine einzige erfirdungsgemässe Vorrichtung für einen Festbettreaktor aus, d.h. in diesem Fall stimmt der querschnitt der Vorrichtung mit demjenigen des Reaktorgehäuses überein.

Es kann auch vorteilhaft sein, mehrere solcher Vorrichtungen im Reaktorgehäuse übereinander anzuordnen, wobei vorteilhaft die übereinanderliegenden Vorrichtungen gegenseitig in bezug auf die Längsachse des Reaktors um ca. 90° gegeneinander versetzt angeordnet sind.

Ein vorteilhaftes Anwandungsgebiet der Erfindung sind sogenannte katalytische Destillationsprozesse, die im wesentlichen in einer Kombination einer katalysierten Reaktion mit einem Destillations- bzw. Rektifikationsprozesse bestehen.

Derartige Prozesse und etliche Anwendungen sind beispielsweise in einem Artikel "CATALYTIC DISTILLATION, Combining chemical reaction with product separation" von William P. Stadig, Houston Regional Editor der Zeitschrift "Chemical Processing" Februar 1987 im einzelnen beschrieben.

Die bekannten Rektifikationskolonnen weisen mindestens einen Festbettreaktor und Stoffaustauschzonen auf, in welchen die bei der Reaktion entstandene Produkte von den unreagierten Edukten und entstandenen unerwünschten Nebenprodukten getrennt werden. Die Rektifikation verläuft kontinuierlich und daher auch in der katalytischen Zone neben der katalysierten Reaktion und zwar vorzugsweise bei Siedetemperatur des zu gewinnenden Produktes, wobei die Siedetemperatur durch eine entsprechende Einstellung des Kolonnendruckes einreguliert wird. Einzelheiten über die Durchführung eines derartigen kombinierten Prozesses finden sich im zitierten Artikel.

In der katalytischen Reaktionszone sind bei dem bekannten Prozess sogenannte Wickelkörper angeordnet. Diese Wickelkörper, die zur Aufnahme eines festen Katalysatormaterials dienen, bestehen aus einem aufgewickelten Band aus Drahtgewebe aus rostfreiem Stahl, mit welchem eine, aus Glasfasern bestehende Schicht verbunden ist, in welcher korrosive Katalysatorpartikel eingebracht sind. Der Vorteil für diese Ausführungsweise wird darin gesehen, dass die Kolonnenwand aus einem korrodierenden Material, wie z.B. kohlehaltigem Stahl ausgeführt sein kann, der nicht mit dem Katakysatormaterial in Berührung kommt.

Ein wesentlicher Nachteil dieser Wickelkörper besteht im Gegensatz zu den erfindungsgemäss angeordneten statischen Mischerstrukturen darin, dass kein radialer Temperatur- und Konzentrationsausgleich über den Kolonnenquerschnitt erfolgt und ausserdem aufgrund der ungeordneten Strömungsform der Reaktanden bzw. der bei der Reaktion entstehenden Produkte nebst der Nebenprodukte ein relativ hoher Druckabfall im Gegensatz zu der geordneten Struktur der erfindungsgemäss eingesetzten statischen Mischerstrukturen entsteht.
Setzt man anstelle der bekannten Wickelkörper die erfindungsgemässen Strukturen in der katalytischen Reaktionszone ein, so werden alle für den vorstehend beschriebenen katalytischen Festbettreaktor erzielten Vorteile erreicht.

Die Erfindung wird im folgenden anhand von in den Zeichnungen schematisch dargestellten Ausführungsformen erläutert.

Es zeigen:
Fig. 1 ein Beispiel einer erfindurgsgemäss ausgebildeten Vorrichtung;
Fig. 1a das Beispiel von Fig.1 in zerlegter Anordnung dargestellt;
Fig. 1b und 1c je eine taschenartige Lage in perspektivischer Darstellungsweise;
Fig. 2a und 2b Details einer varianten Ausführungsform in einer Seitenansicht und in einer Aufsicht;
Fig. 3 einen katalytischen Festbettreaktor;
Fig. 4 eine Rektifikationskolonne zum Durchführen eines kombinierten Prozesses;
Fig. 5 in perspektivischer Darstellung eine weitere Ausführungsform einer erfindungsgemäss ausgebildeten Vorrichtung.

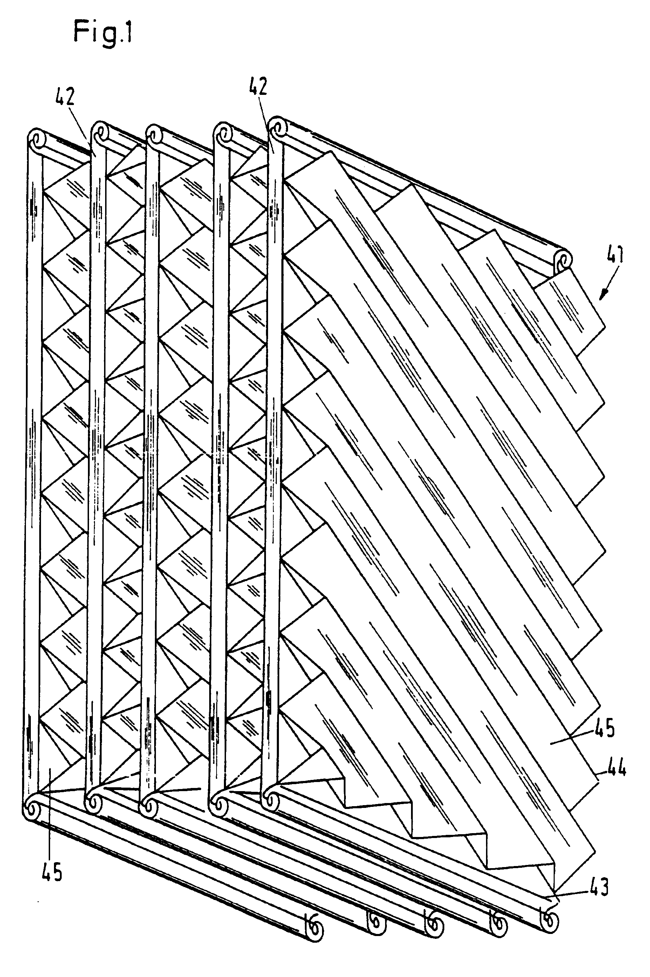

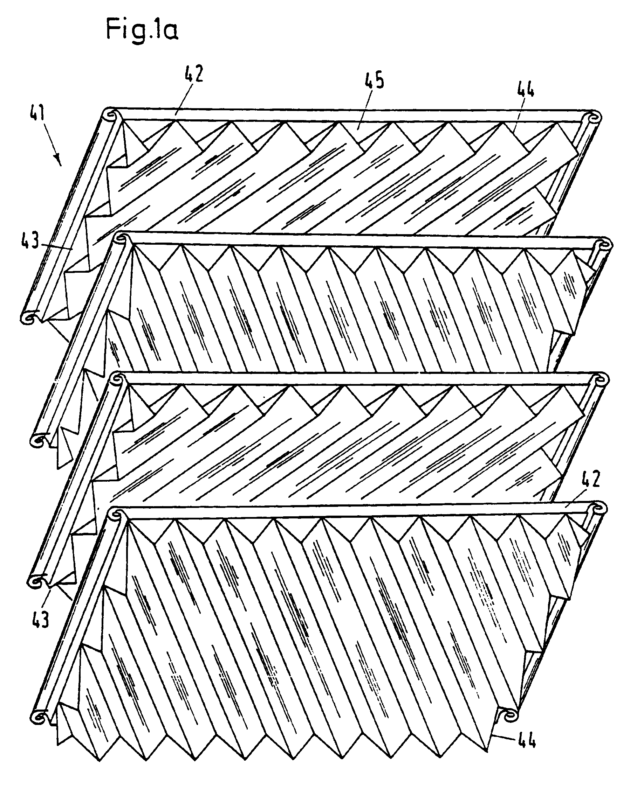

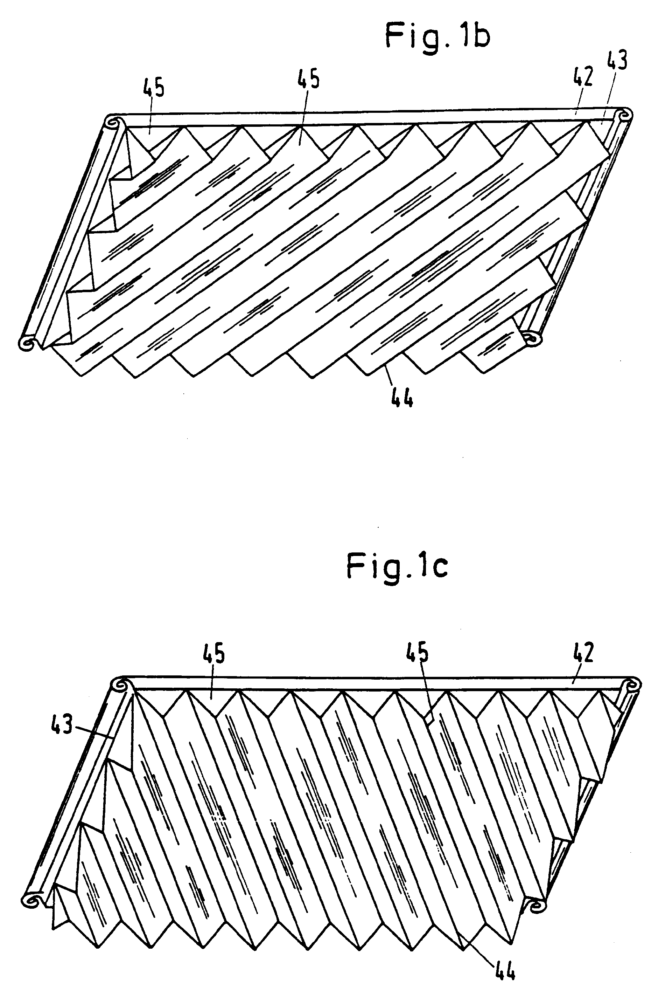

Die in Fig. 1 bzw. in Fig. 1a dargestellte Vorrichtung 41 besteht aus parallel zueinander angeordneten taschenartig ausgebildeten Lagen 42, die jeweils an einer Seitenwand 43 mit Leitelementen 44 verbunden sind.

Diese Leitelemente bestehen im Ausführungsbeispiel aus gewellten Blechplatten, wobei die Wellenberge bzw. -täler Seitenwände der Strömungskanäle 45 für die Reaktanden bilden.

Es ist vorteilhaft, diese gewellten Blechplatten derart auszubilden, dass die von ihnen geformten Strömungskanäle gegen die Längsachse der Taschen 42 einen Winkel einschliessen (vgl. Fig. 1b und 1c).

Benachbarte Lagen werden sodann derart beim Zusammenbau eines Reaktors 41 zusammengefügt, dass sich die Strömungskanäle 45 von benachbarten Taschen 42 kreuzen. Hierdurch wird, insbesondere bei einem, aus mehreren, hintereinander angeordneten, vorzugsweise um ca. 90° gegeneinander versetzten Vorrichtungen 41 eine ausgezeichnete radiale Quervermischung der Teilströme der Reaktenden in Art eines statischen Mischers bewirkt.

Die Leitelemente 44 dienen gleichzeitig zur Verstärkung der mit ihnen z.B. durch Punktschweissung verbundenen Tasche 42. Ihre Wellenberge berühren die Seitenwand der benachbarten Tasche 42 ohne Leitelemente und dienen somit gleichzeitig zur Distanzhaltung. Es sei erwähnt, dass der Begriff "gewellt" auch eine zickzackförmige Profilierung der Platte umfassen soll.

Die Leitelemente 44 können beispielsweise wie im Ausführungsbeispiel aus Blech, auch gelochtem Blech, aber auch aus Kunststoff oder einem Drahtgewebe und dergleichen bestehen, wie beispielsweise in den Unteransprüchen angegeben ist.

Die Seitenwände 43 der Taschen 42, die an ihren seitlichen Rändern miteinanderfest verbunden sind, z.B. durch Niet-, Schweiss- oder Lötverbindungen, bestehen erfindungsgemäss aus einem für die Reaktanden durchlässigen und für das Katalysatormaterial undurchlässigen Material. Beispiele für derartige Materialien sind in den Unteransprüchen angegeben.

In die Taschen 42 wird ein entsprechend dem Verwendungszweck gewähltes Katalysatormaterial 46 in fester Form eingebracht.

Es ist auch möglich, rieselfähiges, mit Kunststoffbasis versetztes Katalysatormaterial in die Zwischenräume einzufüllen und es anschliessend zu polymerisieren, derart, dass eine für die Reaktanden durchlässige, poröse Masse entsteht.

Eine andere Möglichkeit besteht darin, rieselfähiges Katalysatormaterial in die Zwischenräume einzufüllen und anschliessend durch entsprechende Behandlung hieraus eine poröse Keramik- bzw. Sinterstruktur zu bilden.

Im Ausführungsbeispiel weist die Vorrichtung einen kreisförmigen Querschnitt auf. Die Form des Querschnittes wird der jeweils gewählten Formgebung des Reaktorgehäuses angepasst, d.h. sie kann beispielsweise auch quadratisch, rechteckig oder polygonzugartig ausgeführt sein.

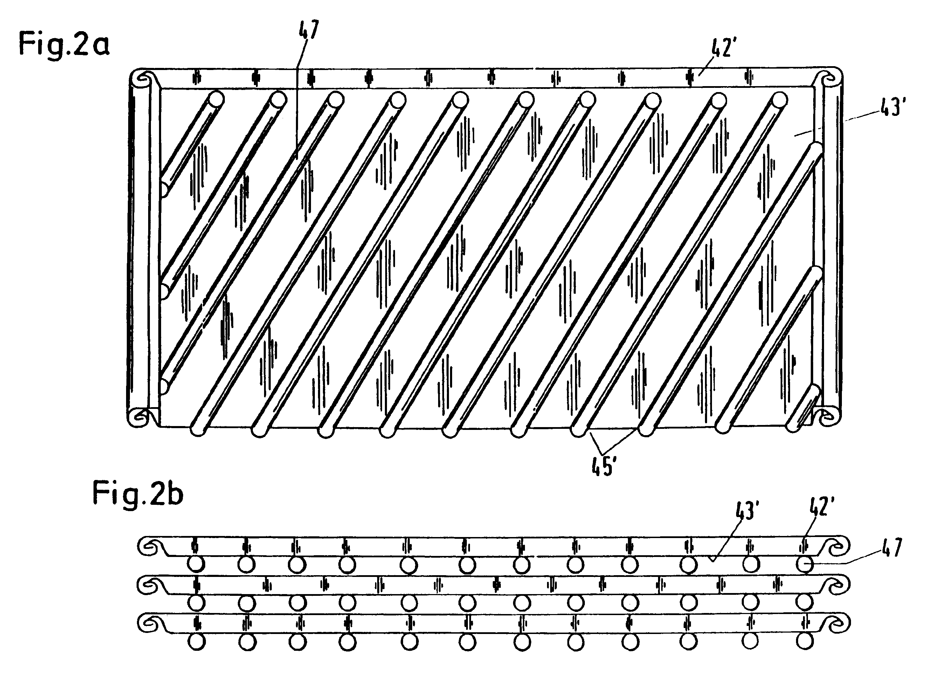

Bei der in den Fig. 2a und 2b dargestellten Ausführungsform sind die Leitelemente der Taschen 42' als Stabelemente 47 ausgebildet und mit den Taschenwänden 43' z.B. durch Punktschweissung verbunden. Im übrigen gelten alle vorstehenden Angaben zu dem in den Fig. 1, 1a, 1b und 1c dargestellten Beispiel der Erfindung.

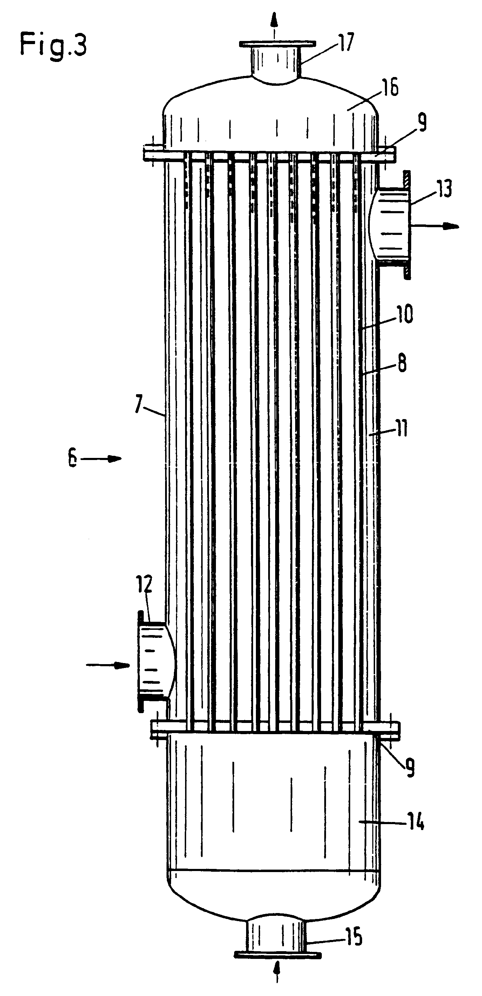

Fig. 3 zeigt in schematischer Darstellungsweise einen katalytischen Festbettreaktor 6 mit einem zylindrischen Gehäuse 7. Im Gehäuse 7 sind Rohre 8 angeordnet, deren offene Enden in Rohrböden 9 befestigt sind.

In den Rohren 8 sind erfindungsgemäss ausgebildete Vorrichtungen 10 übereinander angeordnet, wobei benachbarte Vorrichtungen, wenn sie wie die in Fig. 1, 1a - 1c bzw. Fig. 2a und 2b dargestellten Ausführungsformen ausgebildet sind, jeweils um einen Winkel von 90° gegeneinander bezüglich der Rohrachse versetzt sind.

An den Wärmeaustauschraum 11 sind Rohranschlüsse 12 und 13 angeschlossen für die Zu- und Wegführung eines die Rohre 8 umströmenden wärmeauf- bzw. wärmewegführendes Medium, z.B. eine Salzschmelze.

Unterhalb der Reaktionsrohre 8 ist ein Einlasraum 14 für die durch einen Rohrstutzen 15 zugeführten Reaktanden und oberhalb ein Auslassraum 16 für die Wegführung des Produktes und der Nebenprodukte angeordnet.

Bei einem Beispiel für eine katalysierte Reaktion wird durch den Rohrstutzen 15 und Einlassraum 14 in die Rohre ein Gemisch aus Luft und n-Butan bei einer Temperatur von beispielsweise 200 - 300°C eingeleitet, wobei die Reaktion bei ca. 1 bar durchgeführt werden kann.

Als Katalysator kann in diesem Fall Phosphor- und Vanadiumoxid verwendet werden.

Die Reaktion verläuft exotherm und die hierbei entstehende Wärme wird beispielweise von einer Salzschmelze, die dir Reaktionsrohre 8 umströmt, aufgenommen. In nicht dargestellter weise wird die erwärmte Schmelze durch den Auslass 13 entnommen, wieder auf die erforderliche Temperatur gekühlt und durch den Einlass 12 in den Wärmeaustauschraum 11 rezirkuliert.

Als Produkt wird im vorliegenden Fall Maleinsäureanhydrid gasförmig gewonnen und aus dem Reaktor 6 durch den Auslass 17 einer weiteren Aufbereitung zugeführt, da das Produkt nicht in reiner Form gewonnen wird, sondern ihm noch Edukte, im vorliegenden Fall n-Butan bzw. bei der Bildung von Maleinsäurehydrid entstandene Nebenprodukte, wie Sauerstoff, Wasser, Maleinsäure, Kohlenmonoxid und Kohlendioxid beigemischt sind.

Die weitere Aufbereitung kann in an sich bekannter Weise, beispielsweise in Destillationsprozessen erfolgen.

Aufgrund der Anwendung der erfindungsgemäss ausgebildeten Vorrichtungen und ihrer an vorstehender Stelle beschriebenen Vorteile werden wesentlichweniger Rohre benötigt als bei dem in der Einleitung beschriebenen bekannten Reaktor. Ueberhitzungen werden mindestens weitgehend aufgrund des guten Wärmeübergangs durch die Rohrwandungen vermieden, so dass gegenüber der bekannten Ausführungsform nur relativ geringe Mengen von Kohlenmonoxid, Kohlendioxid und Wasser, welche die Selektivität und Akitivitat des Katalysators herabsetzen, gebildet.

Es sind auch Ausführungsformen eines solchen katalytischen Festbettreaktors möglich, wobei dieser nur aus einem Rohr besteht, in dem mindestens eine erfindungsgemässe Vorrichtung angeordnet ist.

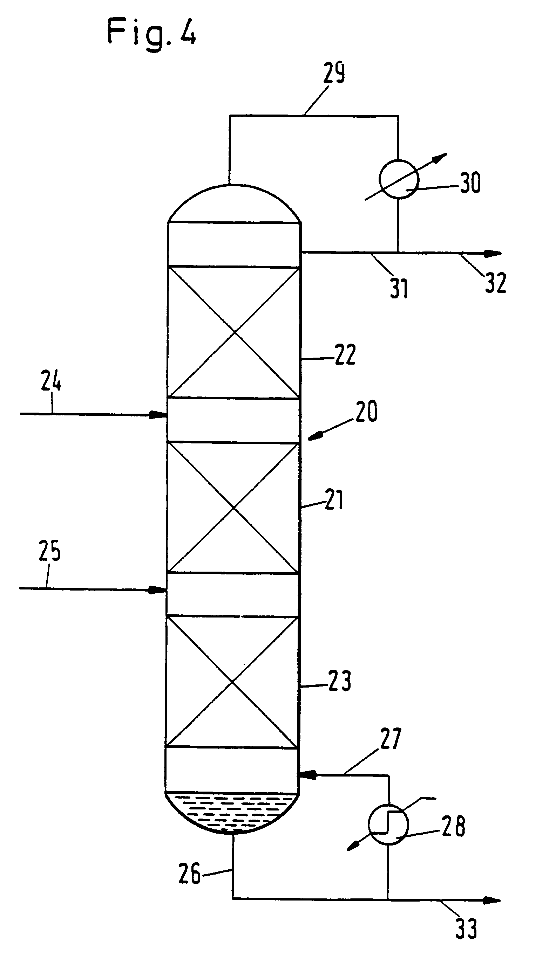

Fig. 4 zeigt in schematischer Darstellungsweise eine katalytische Rektifikationskolonne 20, in welcher ein mit erfindungsgemäss ausgebildeten Vorrichtungen (siehe z.B. Fig. 1) bestückter katalytischer Festbettreaktorabschnitt 21 angeordnet ist.

Ober- und unterhalb dieses Festbettreaktorabschnittes 21 sind Stoffaustauschabschnitte 22 und 23 angeordnet. In letzteren findet die Stofftrennung der im Abschnitt 21 entstandenen Produkte statt. Diese Abschnitte können in bekannter Weise z.B. als Sieb- oder Glockenböden ausgebildet sein. Die Packungskörpe können von flüssigen und gas- bzw. dampfförmigen Phasen im Gegenstrom durchsetzt werden. Die einzelnen gefalteten Lagen können in einschichtig ausgebildet sein, d.h., dass sie dann keine Doppelwände mit Zwischenräumen auf aufweisen.

Ausser der im Katalysatorfeststoffbett 21 stattfindenden Reaktion der im Ausführungsbeispiel durch die Leitungen 24 und 25 eingeleiteten Reaktanden findet in diesem Abschnitt auch eine Rektifikation des Reaktionsgemisches statt.

An den Boden der kolonne ist eine Ableitung 26 für das flüssige Sumpfprodukt angeschlossen. Ein Teil dieses Produktes wird in an sich bekannter Weise durch eine Leitung 27 in die kolonne 20 nach Verdampfung in einem Verdampfer 28 rezirkuliert.

Ebenfalls ist in bekannter Weise am Kopf der kolonne 20 eine Leitung 29 für die Entnahme des gas- bzw. dampfförmigen Kopfproduktes angeschlossen. Nach Verflüssigung dieses Kopfproduktes in einem Kondensator 30 wird eine Teilmenge als Rücklauf durch eine Leitung 31 in die Kolonne zurückgeführt, während die übrige Flüssigkeitsmenge über eine Leitung 32 aus dem Rektifikationsprozess weggeführt wird.

Die Vorteile des erfindungsgemäss ausgebildeten Festbettabschnittes 21 sind die gleichen wie diejenigen des in Fig. 3 dargestellten Ausführungsbeispieles.

Da die Rektifikationskolonne 20 bei Siedetemperatur des sei der Reaktion entstehenden Produktes betrieben wird, entstehen allerdings keine Ueberhitzungssstellen, da bei einer exothermen Reaktion die entstehende Wärme eine Verdampfung der flüssigen Phase bewirkt. Letzteres ist jedoch in diesem Fall in energetischer Hinsicht infolge der geringeren erforderlichen Verdampferleistung der Verdampfers 28 von wesentlichem Vorteil.

Die Anwendung der Erfindung ermöglicht ausserdem die Massstabsvergrösserung derartiger Anlagen aufgrund der vorstehend genannten Vorteile.

Je nach Prozess wird der Feststoffkatalysatorabschnitt 21 im mittleren, unteren oder oberen Teil der Kolonne angeordnet.

Bei dem vorliegenden Ausführungsbeispiel soll aus Methyl- alkohol und einer Mischung aus gesättigten und ungesättigten 4-Karbon Kohlenwasserstoffen Miethyl-tertiär-Butyl- Aether gewonnen werden.

Als Katalysatormaterial im Abschnitt 21 können z.B. saure Ionenaustauschharze verwendet werden.

Der aus Methylalkohol bestehende Reaktand wird durch die Leitung 24 oberhalb des Abschnitts 21 und der aus einer Mischung aus gesättigten und ungesättigten 4-Karbon Kohlenwasserstoffen bestehende Reaktand wird durch die Leitung 25 unterhalb des Abschnitts 21 in die Kolonne 20 eingeleitet.

Aus den Reaktionsprodukten werden im Stoffaustauschabschnitt 22 im Gegenstrom zur flüssigen Phase unreagierte 4-Karbon Kohlenwasserstoffe abgetrennt, die dampfförmig am Kopf der Kolonne 20 entnommen, im Kondensator 30 verflüssigt und zum Teil als Rücklaufwieder in die Kolonne 20 zurückgeführt werden. Die verbleibende Restmenge kann beispielsweise wieder durch Leitung 25 als Reaktand in die Kolonne 20 zurückgeführt werden.

Das in flüssiger Form gewonnene Endprodukt, das aus Methyl-tertiär-Butyl-Aether besteht, wird am Boden der Kolonne 20 aus dieser herausgeführt. Eine Teilmenge wird im Verdampfer 8 verdampft und in die Kolonne 20 rezirkuliert. Durch Leitung 33 wird das flüssige Endprodukt aus der Anlage zur weiteren Aufbereitung entnommen.

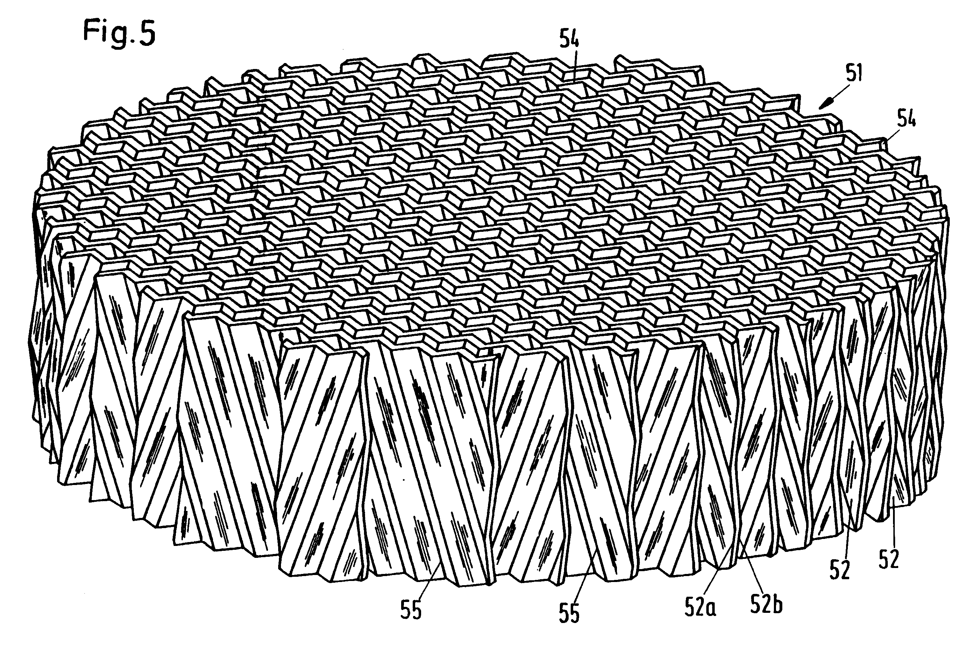

Die in Fig. 5 dargestellte Vorrichtung 51 besteht aus parallel zueinander angeordneten, gefalteten Lagen 52, die jeweils einen Doppelmantel 52a und 52b aufweisen, wobei die Wände aus einem für die Reaktanden durchlässigen und für das Katalysatormaterial undurchlässigen Material bestehen.

In den von den Doppelwänden 52a und 52b gebildeten Zwischenräumen ist ein entsprechend dem Verwendungszweck gewähltes Katalysatormaterial 54 in fester Form eingebracht.

Es ist auch möglich, rieselfähiges, mit Kunststoffbasis versetztes Katalysatormaterial in die Zwischenräume einzufüllen und es anschliessend zu polymerisieren, derart, dass eine für die Reaktanden durchlässige, poröse Masse entsteht.

Eine andere Möglichkeit besteht darin, rieselfähiges Katalysatormaterial in die Zwischenräume einzufüllen und anschliessend durch entsprechende Behandlung hieraus eine poröse Keramik- bzw. Sinterstruktur zu bilden.

Im Ausführungsbeispiel weist die Vorrichtung einen kreisförmigen Querschnitt auf. Die Form des Querschnittes wird der jeweils gewählten Formgebung des Reaktorgehäuses angepasst, d.h. sie kann beispielsweise auch quadratisch, rechteckig oder polygonzugartig ausgeführt sein.

Die einzelnen Lagen 52 sind gefaltet, wobei die Faltungen 55 einen Winkel zur Längsachse der Vorrichtung aufweisen und sich die Faltungen von benachbarten Lagen 52 kreuzen.

## Patentansprüche

1. Vorrichtung (41, 51) zur Durchführung katalysierter Reaktionen, die aus einer Mehrzahl von Lagen (42, 52) besteht, wobei zwischen den benachbarten Lagen Strömungskanäle (45) vorhanden sind, dadurch gekennzeichnet, dass die Lagen (42, 52) eine Struktur mit Doppelwänden (43, 52a, 52b) aufweisen, unter Freilassung von Zwischenräumen zwischen den Doppelwänden, in welche aus Feststoffpartikeln bestehende Katalysatormaterialien (54) eingebracht sind, wobei die Wände (43, 52a, 52b) der Lagen (42, 52) für die Reaktanden durchlässig und für die Katalysatormaterialien undurchlässig sind, wobei die Struktur der Lagen Strömungskanäle (45) bildet, weiche einen Winkel gegen die Längsachse der Vorrichtung (41, 51) einschliessen, und wobei benachbarte Lagen (42, 52) derart zusammengelegt sind, dass sich deren Strömungskanäle (45) kreuzen.

2. Vorrichtung (51) nach Anspruch 1, dadurch gekennzeichnet, dass die ganze Struktur der Lagen (52) unter Freilassung von Zwischenräumen doppelwandig (52a, 52b) ausgeführt ist.

3. Vorrichtung (41) nach Anspruch 1, dadurch gekennzeichnet, dass die Doppelwände (43) der Lagen (42) taschenartig ausgebildet sind, wobei in die Zwischenräume der Taschen Katalysatormaterialien eingebracht sind, und dass die einzelnen Taschen mindestens an einer Wand (43) mit Leitelementen (44) verstärkt sind, welche gleichzeitig Wände der Strömungskanäle (45) bilden.

4. Vorrichtung (41) nach Ansprüche 3, dadurch gekennzeichnet, dass die Leitelemente (44) aus gewellten Platten bestehen.

5. Vorrichtung nach Anspruch 3, dadurch gekennzeichnet, dass die Leitelernente aus Stabelementen (47) bestehen.

6. Vorrichtung nach Anspruch 3, dadurch gekennzeichnet, dass die Leitelemente (44) aus senkrecht zur Taschenebene angebrachten Platten bestehen.

7. Vorrichtung nach einem der vorangehenden Ansprüche, dadurch gekennzeichnet, dass die Wände (43, 52a, 52b) der Lagen aus Drahtgewebe oder Drahtgewirk bestehen.

8. Vorrichtung nach Anspruch 7, dadurch gekennzeichnet, dass die Drähte mindestens zum Teil aus Metall bestehen.

9. Vorrichtung nach Anspruch 7, dadurch gekennzeichnet, dass die Drähte aus Glas- oder Kunststoff bestehen.

10. Vorrichtung nach einem der Ansprüche 1 bis 6, dadurch gekennzeichnet, dass die Wände (43, 52a, 52b) der Lagen aus einem Vlies aus Kunststoff- und/oder Glasfasern bestehen.

11. Vorrichtung nach einem der Ansprüche 1 bis 6, dadurch gekennzeichnet, dass die Wände (43, 52a, 52b) der Lagen aus einem porösen Material bestehen, derart, dass die Poren von einer solchen Grösse sind, dass sie für die Reaktanden durchlässig und für die Katalysatormaterialien undurchlässig sind.

12. Vorrichtung nach einem der Ansprüche 1 bis 6, dadurch gekennzeichnet, dass die Wände (43, 52a, 52b) der Lagen aus einem Sintermaterial bestehen.

13. Vorrichtung nach einem der vorangehenden Ansprüchen, dadurch gekennzeichnet, dass die Feststoffpartikel nach Einfüllung in die Zwischenräume ihren Aggregatzustand z.B. durch Polymerisation ändern, wobei die hierbei entstandene poröse Masse durchlässig für die Reaktanden ist.

14. Vorrichtung nach einem der vorangehenden Ansprüchen, dadurch gekennzeichnet, dass aus den Feststoffpartikeln nach dem Einfüllen in die Zwischenräume eine poröse Keramik- bzw. Sinterstruktur gebildet ist, welche durchlässig für die Reaktanden ist.

15. Verwendung einer Vorrichtung (41, 51) nach einem der vonangehenden Ansprüche in katalytischen Reaktoren (6) oder in katalysierten Rektifikationskolonnen (20).

16. Verwendung einer Vorrichtung (41, 51) nach einem der Ansprüche 1 bis 14 in einem katalytischen Reaktor (6) zum Herstellen von Methyl-Tertiär-Butyl-Aether aus Methylalkohol.

17. Verfahren zum Herstellen von Methyl-Tertiär-Butyl-Aether aus Methylalkohol, bei welchem die Reaktanden mindestens eine Vorrichtung (41, 51) nach einem der Ansprüche 1 bis 14 durchströmen.

## Claims

1. A device (41, 51) for carrying out catalytic reactions, comprising a plurality of layers (42, 52), flow channels (45) being present between the adjoining layers, characterised in that the layers (42, 52) exhibit a structure with double walls (43, 52a, 52b), leaving spaces between the double walls into which catalyst materials comprising solid particles (54) are introduced, the walls (43, 52a, 52b) of the layers (42, 52) being permeable to the reactants and impermeable to the catalyst materials, the structure of the layers forming flow channels (45) which enclose an angle relative to the longitudinal axis of the device (41, 51), and adjoining layers (42, 52) being placed together in such a way that their flow channels (45) cross.

2. A device (51) as claimed in claim 1, characterised in that the entire structure of the layers (52) is constructed with double walls (52a, 52b), leaving spaces.

3. A device (41) as claimed in claim 1, characterised in that the double walls (43) of the layers (42) are pocket-like, catalyst materials being introduced into the spaces in the pockets, and the individual pockets are reinforced on at least one wall (43) with guide elements (44) which simultaneously form walls of the flow channels (45).

4. A device (41) as claimed in claim 3, characterised in that the guide elements (44) comprise corrugated sheets.

5. A device as claimed in claim 3, characterised in that the guide elements comprise rod elements (47).

6. A device (41) as claimed in claim 3, characterised in that the guide elements (44) comprise sheets mounted perpendicularly relative to the plane of the pockets.

7. A device as claimed in any of the preceding claims, characterised in that the walls (43, 52a, 52b) of the layers comprise woven or knitted filaments.

8. A device as claimed in claim 7, characterised in that the filaments consist at least in part of metal.

9. A device as claimed in claim 7, characterised in that the filaments comprise glass or synthetic plastic material.

10. A device as claimed in any of claims 1 to 6, characterised in that the walls (43, 52a, 52b) of the layers comprise a fleece of synthetic plastic and/or glass fibres.

11. A device as claimed in any of claims 1 to 6, characterised in that the walls (43, 52a, 52b) of the layers comprise a porous material, the pores being of such a size as to be permeable to the reactants and impermeable to the catalyst materials.

12. A device as claimed in any of claims 1 to 6, characterised in that the walls (43, 52a, 52b) of the layers comprise sintered material.

13. A device as claimed in any of the preceding claims, characterised in that the solid particles, after introduction into the spaces, change their state of aggregation, e.g. due to polymerisation, the resulting porous material being permeable to the reactants.

14. A device as claimed in any of the preceding claims, characterised in that from the solid particles, after introduction into the spaces, a porous ceramic or sintered structure is formed, which is permeable to the reactants.

15. Use of a device (41, 51) as claimed in any of the preceding claims in catalytic reactors (6) or catalytic rectifying columns (20).

16. Use of a device (41, 51) as claimed in any of claims 1 to 14 in a catalytic reactor (6) for producing methyl tertiary butyl ether from methyl alcohol.

17. A method of producing methyl tertiary butyl ether from methyl alcohol, in which the reactants flow through at least one device (41, 51) as claimed in any of claims 1 to 14.

## Revendications

1. Dispositif (41, 51) pour la conduite de réactions catalysées, qui se compose de plusieurs couches (42, 52), des canaux de circulation (45) existant entre les couches voisines, caractérisé en ce que les couches (42, 52) ont une structure à parois doubles (43, 52a, 52b) qui laisse subsister entre les parois doubles des intervalles dans lesquels des matières catalytiques (54) sont placées, les parois (43, 52a, 52b) des couches (42, 52) étant perméables aux corps réactionnels et imperméables aux matières catalytiques, qui consistent en des particules solides, la structure des couches formant des canaux de circulation (45) qui inscrivent un angle avec l'axe longitudinal du dispositif (41, 51) et les couches voisines (42, 52) étant assemblées de manière que leurs canaux de circulation (45) se croisent.

2. Dispositif (51) selon la revendication 1, caractérisé en ce que la totalité de la structure des couches (52) est réalisée à parois doubles (52a, 52b) de manière à laisser subsister des intervalles.

3. Dispositif (41) selon la revendication 1, caractérisé en ce que les parois doubles (43) des couches (42) sont conformées en poches, des matières catalytiques étant placées dans les intervalles des poches, et en ce que les poches individuelles sont renforcées au moins sur une paroi (43) par des déflecteurs (44) qui forment également les parois des canaux de circulation (45).

4. Dispositif (41) selon la revendication 3, caractérisé en ce que les déflecteurs (44) consistent en des plaques ondulées.

5. Dispositif selon la revendication 3, caractérisé en ce que les déflecteurs consistent en éléments de tiges (47).

6. Dispositif selon la revendication 3, caractérisé en ce que les déflecteurs (44) consistent en des plaques placées perpendiculairement au plan des poches.

7. Dispositif selon l'une des revendications précédentes, caractérisé en ce que les parois (43, 52a, 52b) des couches consistent en des tissus ou des tricots de fils.

8. Dispositif selon la revendication 7, caractérisé en ce que les fils sont au moins en partie en métal.

9. Dispositif selon la revendication 7, caractérisé en ce que les fils sont en verre ou en matière plastique.

10. Dispositif selon l'une des revendications 1 à 6, caractérisé en ce que les parois (43, 52a, 52b) des couches consistent en une toison de fibres de matière plastique et/ou de verre.

11. Dispositif selon l'une des revendications 1 à 6, caractérisé en ce que les parois (43, 52a, 52b) des couches sont en une matière poreuse telle que les pores aient une grandeur telle qu'ils soient perméables aux corps réactionnels et imperméables aux matières catalytiques.

12. Dispositif selon l'une des revendications 1 à 6, caractérisé en ce que les parois (43, 52a, 52b) des couches consistent en une matière frittée.

13. Dispositif selon l'une des revendications précédentes caractérisé en ce que les particules solides déversées dans les intervalles subissent une modification de leur état physique, par exemple par polymérisation, la masse poreuse qui en résulte étant perméable aux corps réactionnels.

14. Dispositif selon l'une des revendications précédentes caractérisé en ce qu'une structure poreuse céramique ou frittée, qui est perméable aux corps réactionnels, est formée à partir des particules solides ayant été déversées dans les intervalles.

15. Utilisation d'un dispositif (41, 51) selon l'une des revendications précédentes dans des réacteurs catalytiques (6) ou dans des colonnes de rectification catalytique (20).

16. Utilisation d'un dispositif (41, 51) selon l'une des revendications 1 à 14 dans un réacteur catalytique (6) de préparation d'éther de méthyle et de tertio-butyle à partir d'alcool méthylique.

17. Procédé de préparation d'éther de méthyle et de tertio-butyle à partir d'alcool méthylique, suivant lequel les corps réactionnels circulent dans au moins un dispositif (41, 51) selon l'une des revendications 1 à 14.
